# EUROPEAN PATENT APPLICATION

(11) **EP 4 098 312 A1**
(43) Date of publication of application: **07.12.2022**
(21) Application number: 21747806.4
(22) Date of filing: 15.01.2021
(51) Int. Cl.: A61M 37/00

(54) **MEDICAL DEVICE, SIGNAL PROCESSING DEVICE, SIGNAL PROCESSING METHOD, AND SIGNAL PROCESSING PROGRAM**

(30) Priority: 27.01.2020 JP 2020010665
(71) Applicant: FURUKAWA ELECTRIC CO., LTD., Chiyoda-ku Tokyo 100-8322 (JP)
(72) Inventor: HIMURA, Atsushi, Tokyo 100-8322 (JP); YAGI, Takeshi, Tokyo 100-8322 (JP); NARA, Kazutaka, Tokyo 100-8322 (JP); SUEMATSU, Katsuki, Tokyo 100-8322 (JP); ARAI, Tsunenori, Tokyo 100-8322 (JP)
(74) Representative: Winter, Brandl - Partnerschaft mbB
(86) International application number: PCT/JP2021/001305
(87) International publication number: WO 2021/153279

(57) **Abstract**

A medical device 1 used for implanting in a body includes a main body 11, a container 12 included in the main body 11, an insulating soft portion 13 that closes an opening of the container 12, an electrically conductive first conductive portion 14 disposed on a bottom of the container 12, and an electrically conductive second conductive portion 15 disposed on the main body 11. The first conductive portion 14 and the second conductive portion 15 are electrically connected to each other.

## Description

### TECHNICAL FIELD

The present disclosure relates to a medical device used for implanting in a body of a subject, a signal processing device which presents information relating to puncture of an injection needle with respect to the medical device, a signal processing method, and a signal processing program.

### BACKGROUND ART

A body-implantable medical device in which a main body of a medical device is implanted into a body is used for charging a medicinal solution into the body. This medical device reduces the burden on patients who need frequent injections for charging a medicinal solution. Such a medical device has a soft portion through which an injection needle is inserted into the main body. This soft portion is made of, for example, silicone rubber or other materials. With the medical device, the injection needle is inserted through the soft portion to inject a medicinal solution from a distal end portion of the injection needle into a container. The medicinal solution injected in the container is transported through a catheter to blood vessels (vein).

Here, generally, the general flow in inserting an injection needle into a medical device is as follows. Step 1: Confirm where the medical device is implanted from above the skin. Step 2: Pierce the injection needle from above the confirmed skin toward the soft portion. Step 3: Inject the medicinal solution from the injection needle into the container.

In particular, when the injection needle is pierced into the soft portion in Step 2, it is difficult to specify the position of the soft portion from the outside of the body by visual recognition.

In order to address this issue, a device has been considered which is configured to apply the technique proposed in Patent Document 1. This medical instrument includes a soft portion having a mixture of a light emitting material and a resin material. The light emitting material emits near-infrared fluorescence when irradiated with near-infrared excitation light. When such a medical instrument is used, by converting the near-infrared fluorescence into visible light, there is a possibility that the position of the soft portion can be specified by visually recognition.

### PRIOR ART DOCUMENTS

### Patent literatures

Patent Document 1: Japanese Patent No. 5958922

### DISCLOSURE OF THE INVENTION

### Problems to be Solved by the Invention

However, even if the technique proposed in Patent Document 1 is applied, it is difficult to present information as to whether or not the distal end portion of the injection needle is located in the container.

It is an object of the present disclosure to provide a medical device that is embedded for use in the body of a subject and presents information relating to a puncture position of an injection needle, a signal processing device that presents information relating to puncture of an injection needle with respect to the medical device, a signal processing method, and a signal processing program.

### Means for Solving the Problems

In order to solve the above-mentioned problems and to attain the object, an exemplary embodiment of the present disclosure provides a medical device used for implanting in a body, including: a main body; a container included in the main body; an insulating soft portion that closes an opening of the container; an electrically conductive first conductive portion disposed on a bottom of the container; and an electrically conductive second conductive portion disposed on the main body, in which the first conductive portion and the second conductive portion are electrically connected to each other.

The medical device according to the present disclosure further includes an injection part to which a catheter is connected, the injection part being configured to inject a liquid in the container into a blood vessel through the catheter.

In the medical device according to the present disclosure, the second conductive portion has a rectangular shape, an annular shape, or a partially notched curved shape.

In order to solve the above-mentioned problems and to attain the object, an exemplary embodiment of the present disclosure provides a signal processing device including: a signal input unit that inputs an electric signal to an electrically conductive injection needle; a receiving unit that receives an electric signal outputted from a distal end portion of the injection needle inserted into a body via an electrically conductive third conductive portion disposed at a location on a body surface, the location being determined based on a position of the second conductive portion of the medical device; and a presenting unit that presents information based on the electric signal received by the receiving unit.

In the signal processing device according to the present disclosure, the presenting unit presents a magnitude of the electric signal received by the receiving unit.

In the signal processing device according to the present disclosure, the receiving unit receives, via the second conductive portion, an electric signal generated when the injection needle and the first conductive portion are electrically connected to each other.

In the signal processing device according to the present disclosure, the presenting unit includes a determination unit that determines whether the distal end portion of the injection needle is included in the container or not based on the electric signal received by the receiving unit, and a notification unit that notifies a determination result by the determination unit.

The signal processing device according to the present disclosure further includes a storage unit that stores data indicating a change in an electric signal during a time when the injection needle, to which the electric signal is inputted by the signal input unit, enters a body from an outside of the body, passes through the soft portion, and reaches an inside of the container, in which the determination unit refers to the data stored in the storage unit, and determines whether the injection needle is included in the container or not based on the electric signal received by the receiving unit.

In the signal processing device according to the present disclosure, the data stored in the storage unit includes a change in a magnitude of an electric signal until the injection needle reaches inside the container and the distal end portion of the injection needle comes into contact with the first conductive portion.

In order to solve the above-mentioned problems and to attain the object, an exemplary embodiment of the present disclosure provides a signal processing device including: a signal input unit that inputs an electric signal to an electrically conductive injection needle; a receiving unit that receives an electric signal outputted from a distal end portion of the injection needle inserted into a body via an electrically conductive third conductive portion disposed at a predetermined location outside the body; and a presenting unit that presents information based on the electric signal received by the receiving unit.

In order to solve the above-mentioned problems and to attain the object, an exemplary embodiment of the present disclosure provides a signal processing method including: a signal inputting step including inputting an electric signal to an electrically conductive injection needle; a receiving step including receiving an electric signal outputted from a distal end portion of the injection needle; and a presenting step including presenting information based on the electric signal received in the receiving step.

In the signal processing method according to the present disclosure, the presenting step further includes a determining step including referring to data which is stored in a storage unit and indicates a change in an electric signal during a time when the injection needle, to which the electric signal is inputted by the signal inputting step, passes through a soft portion of a medical device and reaches inside of a container, and determining whether the injection needle is included in the container or not based on a change in a magnitude of the electric signal measured in the receiving step, and a notifying step including notifying a determination result by the determining step.

In order to solve the above-mentioned problems and to attain the object, an exemplary embodiment of the present disclosure provides a signal processing program for executing a computer including: a signal inputting step including inputting an electric signal to an electrically conductive injection needle; a receiving step including receiving an electric signal outputted from a distal end portion of the injection needle; and a presenting step including presenting information based on the electric signal received in the receiving step.

### Effects of the Invention

According to the present disclosure, it is possible to present information relating to puncture of an injection needle.

### BRIEF DESCRIPTION OF THE DRAWINGS

FIG. 1 is a schematic diagram showing a schematic configuration of a medical device implanting in a body and a signal processing device according to an exemplary first embodiment of the present disclosure.
FIG. 2 is a diagram showing a configuration of a medical device.
FIG. 3 is a diagram schematically showing a first example of the shape of a second conductive portion.
FIG. 4 is a diagram schematically showing a second example of the shape of the second conductive portion.
FIG. 5 is a diagram schematically showing a third example of the shape of the second conductive portion.
FIG. 6 is a diagram showing a configuration of a presenting unit.
FIG. 7 is a diagram schematically showing an example of a first change in the level of an electric signal received by a receiving unit.
FIG. 8 is a diagram schematically showing an example of a second change in the level of the electric signal received by the receiving unit.
FIG. 9 is a diagram schematically showing a representative example of a path until an electric signal outputted from an injection needle is transmitted to a third conductive portion when the distal end portion of the injection needle is inserted into a living body.
FIG. 10 is a diagram schematically showing a representative example of a path until an electric signal outputted from the injection needle is transmitted to the third conductive portion when the distal end portion of the injection needle is inserted into a soft portion.
FIG. 11 is a diagram schematically showing a representative example of a path until an electric signal inputted to the injection needle is transmitted to the third conductive portion when the distal end portion of the injection needle is inserted into the container.
FIG. 12 is a diagram schematically showing a representative example of a path until an electric signal inputted to the injection needle is transmitted to the third conductive portion when the distal end portion of the injection needle contacts the first conductive portion.
FIG. 13 is a schematic diagram showing a schematic configuration of a medical device implanting in a body and a signal processing device according to an exemplary second embodiment of the present disclosure.
FIG. 14 is a flowchart for explaining a signal processing method.
FIG. 15 is a flowchart for explaining the presenting step according to step ST3.
FIG. 16 is a diagram showing a configuration of a computer. FIG. 17 is a diagram showing a configuration of a processing circuit.

### PREFERRED MODE FOR CARRYING OUT THE INVENTION

Hereinafter, exemplary embodiments for carrying out the present disclosure will be described in detail with reference to the accompanying drawings. It should be noted that the present disclosure is not limited to the following exemplary embodiments. In addition, the drawings referred to in the following description are merely schematic representations of the shape, size, and positional relationship to the extent that the content of the present disclosure can be understood. That is, the present disclosure is not limited to the shapes, sizes, and positional relationships illustrated in the drawings. Furthermore, in the following description, a medical device which is implanted and used in a living body of a subject including a person and an animal, a signal processing device which presents information relating to puncture of an injection needle with respect to the medical device, a signal processing method, and a signal processing program will be described in detail.

### (First Embodiment)

### <Configuration of Medical Device>

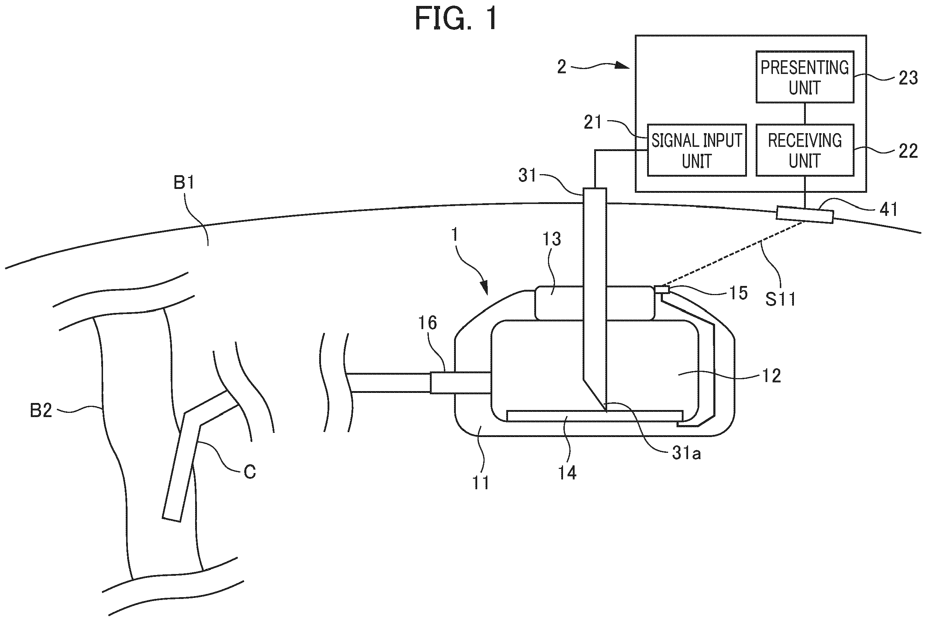

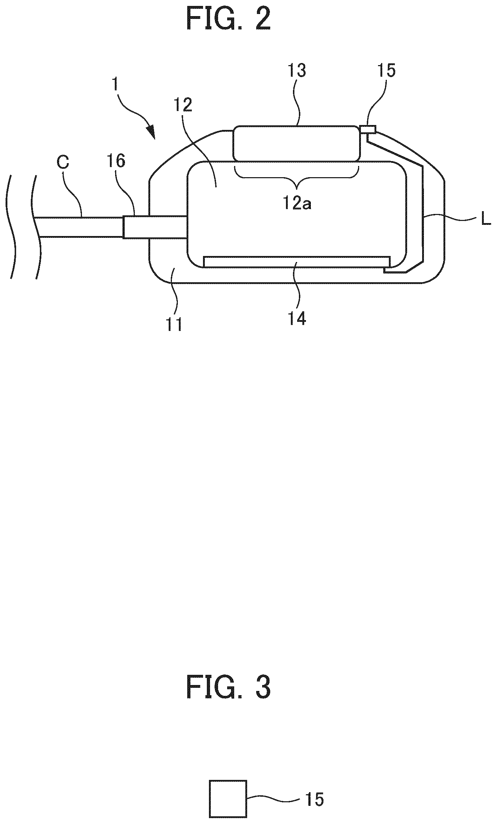

FIG. 1 is a schematic diagram showing a schematic configuration of a medical device 1 and a signal processing device 2 according to the first embodiment. FIG. 2 is a diagram showing a configuration of the medical device 1. The medical device 1 is referred to as, for example, a subcutaneous implantable type port (CV port). The medical device 1 is implanted in a patient for use. FIG. 1 schematically shows a state in which the medical device 1 is implanted in a living body B1 of a patient.

The medical device 1 implanted in a patient's body for use, as shown in FIG. 2, includes a main body 11, a container 12 included in the main body 11, an insulating soft portion 13 (septum) that closes an opening 12a of the container 12, an electrically conductive first conductive portion 14 disposed on a bottom of the container 12, and an electrically conductive second conductive portion 15 disposed on the main body 11. The first conductive portion 14 and the second conductive portion 15 are electrically connected to each other. Furthermore, the medical device 1 further includes an injection part 16 to which a catheter C is connected. The injection part 16 injects the liquid in the container 12 into the blood vessel B2 (vein) via the catheter C.

The main body 11 is, for example, a housing made of epoxy resin or other materials. The container 12 is a tank that temporarily stores a medicinal solution. The container 12 has the opening 12a which has a circular shape on the outside of the main body. FIG. 2 shows that the opening 12a is closed by the soft portion 13.

The soft portion 13 is referred to as a septum. The soft portion 13 closes the opening 12a of the container 12. The soft portion 13 is, for example, a soft lid body (silicone diaphragm) made of silicone rubber, and is provided so as to be exposed from the main body 11 as well. Furthermore, the soft portion 13 has a columnar shape. However, it may have a shape other than a columnar shape. The soft portion 13 is a portion through which an injection needle 31 for the injection (infusion) of a medicinal solution can be inserted from a body surface of a subject after the main body 11 is implanted within the living body B1. It should be noted that FIGS. 1 and 2 each schematically show the external shape of the medical device 1. Therefore, various shapes can be given as the shapes of the main body 11, the container 12, and other components. The injection needle 31 is, for example, a Huber needle. A medicinal solution or the like is discharged from the distal end portion 31a of the injection needle 31.

The first conductive portion 14 is made of a conductive material. Examples of the conductive material include, for example, conductive polymers and metals. In FIGS. 1 and 2, the first conductive portion 14 shows a state of being disposed so as to cover the entire bottom surface of the container 12. However, this is merely an example. In the present disclosure, various shapes and arrangement positions of the first conductive portion 14 can be given.

The second conductive portion 15 is made of a conductive material. Examples of the conductive material include, for example, conductive polymers and metals. The second conductive portion 15 may be embedded within the main body 11. Alternatively, the second conductive portion 15 may be exposed on the surface of the main body 11. It should be noted that, when the second conductive portion 15 is exposed on the surface of the main body 11, the second conductive portion 15 preferably includes a material that does not affect the living body. It should also be noted that, in a case in which the second conductive portion 15 is exposed on the surface of the main body 11, a connective tissue is formed between the second conductive portion and the body tissue when the second conductive portion 15 is left in the living body B1 for a long period of time. This leads to electrical stability. As a result, there is little possibility that the determination criterion by the signal processing device 2 changes. Furthermore, there is a small possibility that the electric signal outputted from the second conductive portion 15 cannot be detected by the signal processing device 2.

The first conductive portion 14 and the second conductive portion 15 are electrically connected to each other. FIG. 1 shows an example of the first conductive portion 14 and the second conductive portion 15 being electrically connected by a connection line L. However, the present disclosure is not limited thereto. For example, when the main body 11 is made of a conductive material, the first conductive portion 14 and the second conductive portion 15 are electrically connected with each other without having a connection by the connection line L.

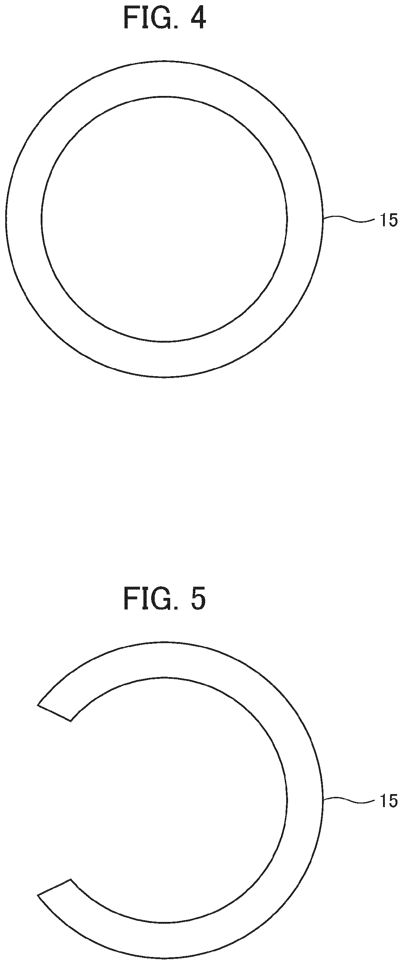

FIGS. 3, 4, and 5 are diagrams each schematically showing the shape of the second conductive portion 15. As shown in FIG. 3, the second conductive portion 15 may have a rectangular shape. As shown in FIG. 4, the second conductive portion 15 may have an annular shape. As shown in FIG. 5, the second conductive portion 15 may have a partially notched curved shape. The shape of the second conductive portion 15 is not limited to the shape shown in FIGS. 3, 4, and 5.

When the second conductive portion 15 has a rectangular shape, it is possible to reduce the size of the second conductive portion 15. Therefore, it is possible to install the second conductive portion 15 without greatly changing the design of the medical device 1.

When the second conductive portion 15 has an annular shape, it is effective when determining the position for placing a third conductive portion 41 to be described later. After the medical device 1 is implanted into the living body B1, the medical device 1 cannot be visually viewed from outside the living body B1 (although the rough position of the medical device 1 can be grasped). Therefore, it becomes difficult to identify where the second conductive portion 15 is disposed. When the second conductive portion 15 has an annular shape, it is not necessary to pinpoint the position where the second conductive portion 15 is disposed. Furthermore, it is possible to easily determine the position of placing the third conductive portion 41.

In a case in which the second conductive portion 15 has a curved shape in which a portion of the second conductive portion 15 is notched, for example, when the external environment is a high magnetic field, it is possible, for example, to reduce the heat generation by the second conductive portion 15 due to the phenomenon of induction heating.

Furthermore, the third conductive portion 41 may have a rectangular shape, a partially notched curved shape, or an annular shape.

The catheter C has one end and another end as shown in FIG. 1. The one end of the catheter C communicates with the injection part 16 of the container 12. The other end of the catheter C is inserted into a blood vessel (vein). This catheter C transports the medicinal solution temporarily stored in the container 12 to the blood vessel.

The medical device 1 configured as described above transmits the electric signal from the first conductive portion 14 to the second conductive portion 15, the electric signal being outputted from the distal end portion 31a of the injection needle 31. Therefore, it is possible to present information relating to puncture of the injection needle 31 by the signal processing device 2 described later. It should be noted that the information relating to puncture of the injection needle 31 refers to a concept including information as to whether or not the distal end portion 31a of the injection needle 31 is entered in the container 12.

### <Configuration and Operation of Signal Processing Device According to First Embodiment>

Next, the configuration and operation of the signal processing device 2 according to the first embodiment will be described with reference to FIG. 1. The signal processing device 2 includes a signal input unit 21, a receiving unit 22, and a presenting unit 23. The signal input unit 21 inputs an electric signal to the conductive injection needle 31. The receiving unit 22 receives the electric signal outputted from the distal end portion 31a of the injection needle 31 inserted into the body via the conductive third conductive portion 41. The conductive third conductive portion 41 is disposed at a location on the body surface (outside the living body B1), which is determined based on the position of the second conductive portion 15 of the medical device 1. The presenting unit 23 presents information based on the electric signal received by the receiving unit 22.

The signal input unit 21 generates a predetermined electric signal. The signal input unit 21 inputs the generated electric signal to the injection needle 31. For example, the signal input unit 21 and the injection needle 31 may be electrically connected by a sterile clip or the like. In addition, as will be described in detail later, there is a possibility that an electric signal (background current) inputted from the signal input unit 21 to the injection needle 31 flows through the living body B1 to reach the vicinity of the heart. Therefore, it is necessary to ensure safety against electric shock. The frequency of the power supply for driving the signal processing device 2 is preferably an electric shock dead band (e.g., several kHz or more). In addition, it is preferable to use 1 µA or less, which is an electric shock safety criterion, in consideration of a case where an electric signal outputted from the injection needle 31 flows in the living body B1 and diverts into an organ (particularly, a heart or the like). Furthermore, when the signal processing device 2 receives the supply of power from a commercial power source, it is preferable to use a CF-type device. The CF type is a device with a mounting part of the F (floating) type. The CF-type device differs from B-type device. The CF-type device has a structure in which an mounted part of the patient is electrically isolated from other parts of the device so that an unacceptable leakage current does not flow between the mounted part of the patient and the ground. Furthermore, the predetermined electric signal used in the signal input unit 21 refers to an AC signal. Furthermore, the frequency of the AC signal is, for example, in the range of 10 kHz to 1 MHz. However, in practice, the frequency of the AC signal is determined as an appropriate frequency in consideration of the range corresponding to a dead zone for a living body and safety standards.

The third conductive portion 41 is made of a conductive material. Examples of the conductive material include conductive polymers and metals. The third conductive portion 41 is preferably located near the location where the second conductive portion 15 of the medical device 1 implanted in the body is located.

The receiving unit 22 is electrically connected to the third conductive portion 41. The receiving unit 22 receives an electric signal from the third conductive portion 41. Although details will be described later, the presenting unit 23 presents information based on the electric signal received by the receiving unit 22.

According to such a configuration, the signal processing device 2 can present information relating to whether or not the distal end portion 31a of the injection needle 31 is included in the container 12 of the medical device 1 for a user who punctures the medical device 1 with the injection needle 31. The user is, for example, a person who pierces the injection needle 31 and injects the medicinal solution into the medical device 1. The user is specifically a medical doctor, a nurse, or the like.

The presenting unit 23 presents the magnitude of the electric signal received by the receiving unit 22. The presenting unit 23, for example, may indicate the magnitude of the electric signal received by the receiving unit 22 as the movement of the needle in an analog fashion. Alternatively, the presenting unit 23 may indicate such magnitude as a digital numerical value. The user punctures with the injection needle 31, and can determine whether or not the distal end portion 31a of the injection needle 31 is included in the container 12 while visually observing the result presented by the presenting unit 23.

According to such a configuration, when the user punctures with the injection needle 31, it is possible for the signal processing device 2 to determine whether or not the distal end portion 31a of the injection needle 31 is included in the container 12 of the medical device 1 based on the information presented by the presenting unit 23.

The receiving unit 22 receives an electric signal generated when the injection needle 31 and the first conductive portion 14 are electrically connected to each other, via the second conductive portion 15. The electric signal generated encompasses an electric signal having a large amplitude (the amount of current in the case of a DC current), and the like. Here, a flow for receiving an electric signal generated when the injection needle 31 and the first conductive portion 14 are electrically connected by the receiving unit 22 will be described. In the following description, an electric signal is input from the signal input unit 21 to the injection needle 31.

When the living body B1 is punctured with the injection needle 31 from the outside, and the distal end portion 31a of the injection needle 31 comes into contact with the first conductive portion 14 disposed on the bottom surface of the container 12, an electric signal is supplied from the distal end portion 31a of the injection needle 31 to the first conductive portion 14. The electric signal supplied to the first conductive portion 14 is supplied to the second conductive portion 15. The electric signal supplied to the second conductive portion 15 is supplied to the third conductive portion 41 via the path S11 in the living body B1 having conductivity. The receiving unit 22 receives the electric signal supplied to the third conductive portion 41. It should be noted that FIG. 1 schematically shows a representative example of the path S11. Accordingly, the present disclosure is not limited thereto. It should be noted that the distal end portion 31a of the injection needle 31 and the first conductive portion 14 may be electrically connected to each other. Therefore, the first conductive portion 14 may be buried below the bottom surface of the container 12 in the main body 11. In the present embodiment, the "first conductive portion 14 disposed on the bottom surface of the container 12" includes a case where the first conductive portion 14 is disposed directly on the bottom surface of the container 12. In this case, the first conductive portion 14 is exposed on the bottom surface of the container 12. Furthermore, the "first conductive portion 14 disposed on the bottom surface of the container 12" includes a case where the first conductive portion 14 is disposed inside the bottom surface of the container 12. In this case, it refers to a case where the first conductive portion 14 is embedded in the interior of the bottom surface of the container 12.

According to such a configuration, the signal processing device 2 presents, by the presenting unit 23, that the electric signal is received by the receiving unit 22. Thus, it is possible for the signal processing device 2 to explicitly indicate that the distal end portion 31a of the injection needle 31 has contacted the first conductive portion 14 disposed on the bottom surface of the container 12.

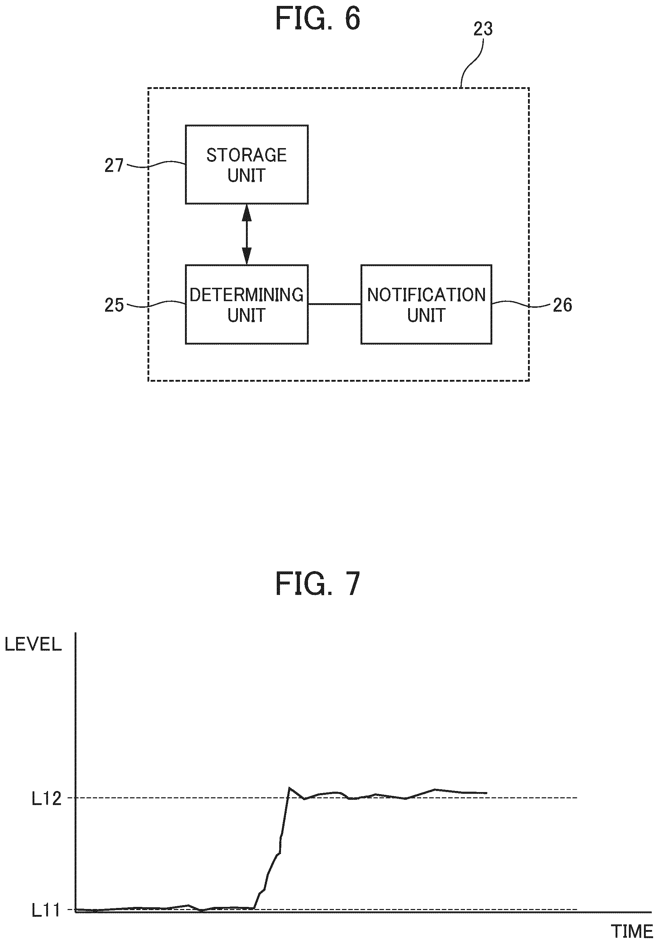

FIG. 6 is a diagram showing a configuration of the presenting unit 23. The presenting unit 23, as shown in FIG. 6, includes a determination unit 25 and a notification unit 26. The determination unit 25 determines whether the distal end portion of the injection needle 31 is included in the container 12 or not based on the electric signal received by the receiving unit 22. The notification unit 26 notifies the determination result by the determination unit 25.

FIG. 7 is a diagram schematically showing a first change in the level of the electric signal received by the receiving unit 22. The level of the electric signal indicates the magnitude of the electric signal. The level of the electric signal relates, for example, to current, voltage, frequency, and intensity. The level L11 shown in FIG. 7 indicates the level of the electric signal in a state where the injection needle 31 is located outside the body. The level L12 shown in FIG. 7 indicates the level of the electric signal in a state where the distal end portion 31a of the injection needle 31 is in contact with the first conductive portion 14. The level of the electric signal of each state shown in FIG. 7 is an example. Accordingly, the present disclosure is not limited thereto.

The determination unit 25 determines whether or not the distal end portion of the injection needle 31 is included in the container 12 based on the level of the electric signal received by the receiving unit 22. More specifically, when the electric signal received by the receiving unit 22 indicates the level L11, the determination unit 25 determines that the injection needle 31 is located outside the body and that the distal end portion of the injection needle 31 is not included in the container 12. Furthermore, when the electric signal received by the receiving unit 22 indicates the level L12, the determination unit 25 determines that the distal end portion of the injection needle 31 is included in the container 12. It should be noted that the determination unit 25 may determine whether the distal end portion of the injection needle 31 is included in the container 12 or not based on the difference (change) between the level L11 and the level L12. Furthermore, the determination unit 25 may determine whether the distal end portion of the injection needle 31 is included in the container 12 or not based on the amplitude of the electric signal instead of the determination based on the level of the electric signal.

The notification unit 26 includes, for example, a light emitting unit or a display unit. The light emitting unit indicates the result of the determination by the determination unit 25 by way of the light emitting state. The display unit displays the result of the determination by the determination unit 25 by way of a message. The light emitting unit is, for example, an LED. In a case where the notification unit 26 has a light emitting unit, the light emitting unit is turned off when the distal end portion 31a of the injection needle 31 is not in contact with the first conductive portion 14. When the distal end portion 31a of the injection needle 31 comes into contact with the first conductive portion 14, the light emitting unit emits light. The user can determine whether the distal end portion 31a of the injection needle 31 is in contact with the first conductive portion 14 or not based on the light emission state of the notification unit 26. In other words, the user can determine whether the distal end portion 31a of the injection needle 31 is entered in the container 12 or not by the light emission state of the notification unit 26.

It should be noted that the light emission state is not limited to two types, i.e., the off state and the on state of the light emission state. The light emission state may be changed in multiple stages in accordance with the distance between the distal end portion 31a of the injection needle 31 and the first conductive portion 14. In the case of such a configuration, the user can grasp the details up to when the distal end portion 31a of the injection needle 31 comes into contact with the first conductive portion 14. As a result, the user can adjust the speed, strength, and the like when the puncture with the injection needle 31 is performed.

The display unit is, for example, a display. In case that the notification unit 26 includes the display unit, when the distal end portion 31a of the injection needle 31 is not in contact with the first conductive portion 14, the display unit displays a message such as "the distal end portion of the injection needle has not yet reached the container", and when the distal end portion 31a of the injection needle 31 comes into contact with the first conductive portion 14, the display unit displays a message such as "the distal end portion of the injection needle has reached the container". It is possible for the user to determine whether the distal end portion 31a of the injection needle 31 is included in the container 12 or not based on the display state of the notification unit 26.

As shown in FIG. 6, the presenting unit 23 includes a storage unit 27. The storage unit 27 stores data indicating a change in the electric signal. This data shows the change in the electric signal during the time when the injection needle 31, to which the electric signal is inputted by the signal input unit 21, enters the body from the outside of the body, passes through the soft portion 13, and reaches the inside of the container 12. Furthermore, the data stored in the storage unit 27 may include a change in the magnitude of the electric signal. Here, the change in the magnitude of the electric signal refers to the change in the magnitude of the electric signal until the injection needle 31 reaches the inside of the container 12 and the distal end portion 31a of the injection needle 31 comes into contact with the first conductive portion 14. The storage unit 27 stores, for example, data of the waveform shown in FIG. 7. The determination unit 25 refers to the data stored in the storage unit 27. Then, the determination unit 25 determines whether the injection needle 31 is included in the container 12 or not based on the electric signal received by the receiving unit 22.

Here, in the present invention, the second conductive portion 15 provided outside the main body 11 is designed such that the impedance of the path between the injection needle 31, the first conductive portion 14, the second conductive portion 15, and the third conductive portion 41 is smaller than the impedance of the path between the injection needle 31 and the third conductive portion 41 for the resistance (impedance) seen from the third conductive portion 41 which is the body surface electrode, in a state in which the injection needle 31 is pierced into the skin until just before the surface of the soft portion 13. For example, by changing the surface area and the place to be disposed of the second conductive portion 15, or by changing the surface area and the place to be disposed of the third conductive portion 41, it is possible to achieve the design described above. Examples of methods of differentiating the impedance according to the path through which the electric signal is transmitted may include a method of insulating the outside of the injection needle 31 (other than the distal end portion 31a).

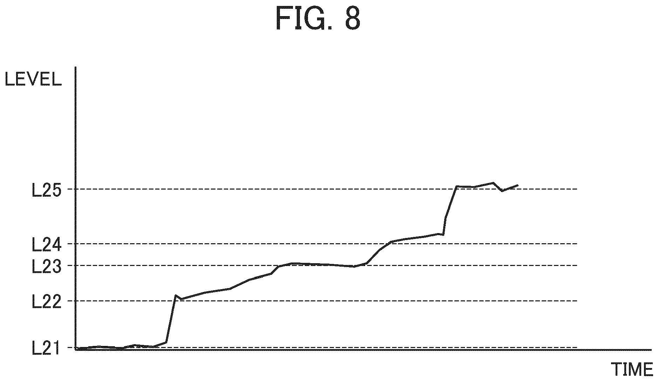

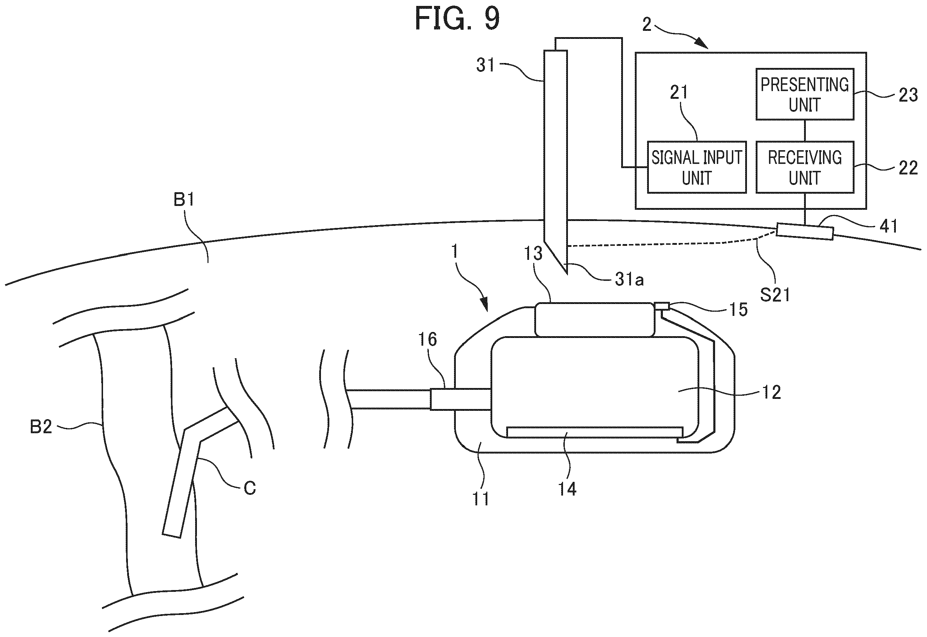

FIG. 8 is a diagram schematically showing a second change in the level of the electric signal received by the receiving unit 22. The level L21 shown in FIG. 8 indicates the level of the electric signal when the injection needle 31 is located outside the body. The level L22 shown in FIG. 8 indicates the level of the electric signal when the distal end portion 31a of the injection needle 31 is inserted into and passes through the living body B1. As the injection needle 31 is inserted deeper into the living body B1, the contact area between the injection needle 31 and the living body B1 becomes larger. Therefore, the path to be transmitted to the third conductive portion 41 is increased. Thus, the level of the electric signal is assumed to gradually increase. FIG. 9 is a diagram schematically showing a representative example of a path S21. The path S21 is a path through which the electric signal outputted from the injection needle 31 is transmitted to the third conductive portion 41 when the distal end portion 31a of the injection needle 31 is inserted into the living body B1.

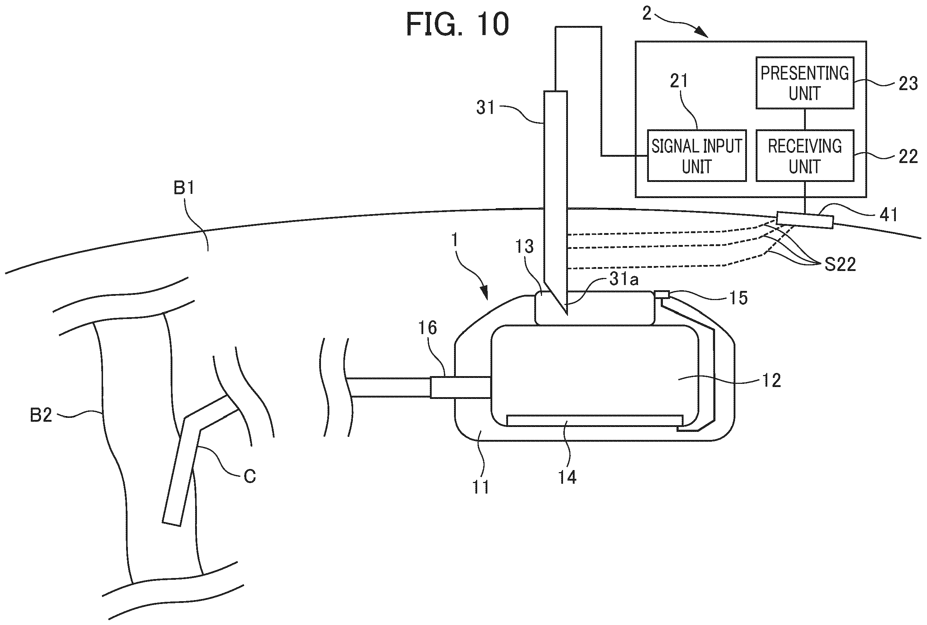

The level L23 shown in FIG. 8 indicates the level of the electric signal when the distal end portion 31a of the injection needle 31 is inserted into the soft portion 13 and is passing through the soft portion 13. FIG. 10 is a diagram schematically showing a representative example of paths S22. The paths S22 are paths through which the electric signal outputted from the injection needle 31 is transmitted to the third conductive portion 41 when the distal end portion 31a of the injection needle 31 is inserted into the soft portion 13. It should be noted that, in FIG. 10, a plurality of paths S22 are shown. This indicates that as the injection needle 31 is inserted deeper into the living body B1, the contact area between the injection needle 31 and the living body B1 becomes larger, leading to the increase of the paths transmitting to the third conductive portion 41.

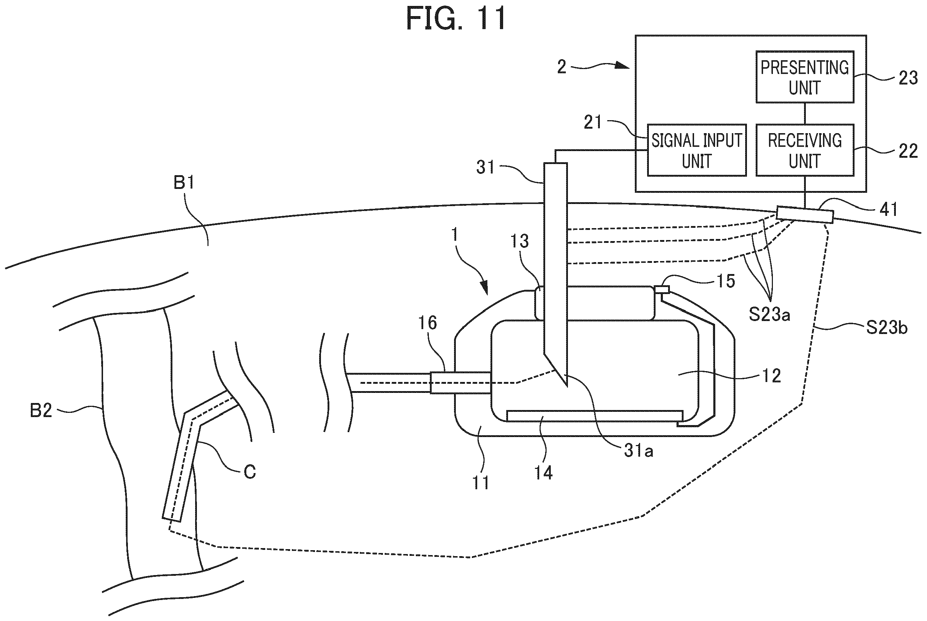

The level L24 shown in FIG. 8 indicates the level of electric signal when the distal end portion 31a of the injection needle 31 is inserted into and passing through the container 12. FIG. 11 is a diagram schematically showing a representative example of paths S23a and S23b. The paths S23a and S23b refer to paths through which electric signals inputted to the injection needle 31 are transmitted to the third conductive portion 41 when the distal end portion 31a of the injection needle 31 is inserted into the container 12. Furthermore, the container 12 and the catheter C are filled with a saline solution, a portion of blood, or the like. Therefore, the container 12 and the catheter C have conductivity.

Here, a saline solution which fills the inside of the container 12 will be described. In the interior of the container 12, a saline solution is usually enclosed for maintenance. Thus, a certain degree of electrical conduction is also retained in the container 12. In this embodiment, for example, a sugar solution may be used instead of the saline solution. In this embodiment, a sugar solution may be added to the saline solution. Alternatively, in this embodiment, an X-ray contrast medium may be added to the saline solution. The X-ray contrast medium is a nonionic contrast medium, for example, iopamidol. The reason for this will be described below. When the injection needle 31 enters into the container 12, it is assumed that the electrical current value gradually increases. It is also assumed that, when the distal end portion 31a of the injection needle 31 comes into contact with the first conductive portion 14 disposed at the bottom of the container 12, the electrical current value becomes the highest. Here, since the saline solution has a certain degree of electrical conductivity, there is a possibility that a change in the electrical current value at the moment when the distal end portion 31a of the injection needle 31 comes into contact with the first conductive portion 14 becomes small. Therefore, when the container 12 is filled with a sugar solution having a lower conductivity (a high resistivity liquid), a saline solution to which a sugar solution is added, or a saline solution to which an X-ray contrast medium is added, it is possible to clearly show the change in the electrical current value at the moment when the distal end portion 31a of the injection needle 31 comes into contact with the first conductive portion 14.

The resistivity of the skin is about several 10 Ωm. The resistivity of the muscle is about 2 to 7 Ωm. The resistivity of the fascia between the skin and the muscle is about 100 Ωm or less. The resistivity of the vessel is about 1.4 Ωm. The resistivity of the soft portion 13 is about 1000 Ωm. When a sugar solution is used instead of a saline solution in the container 12, the resistivity of the sugar solution is about 10 kΩm.

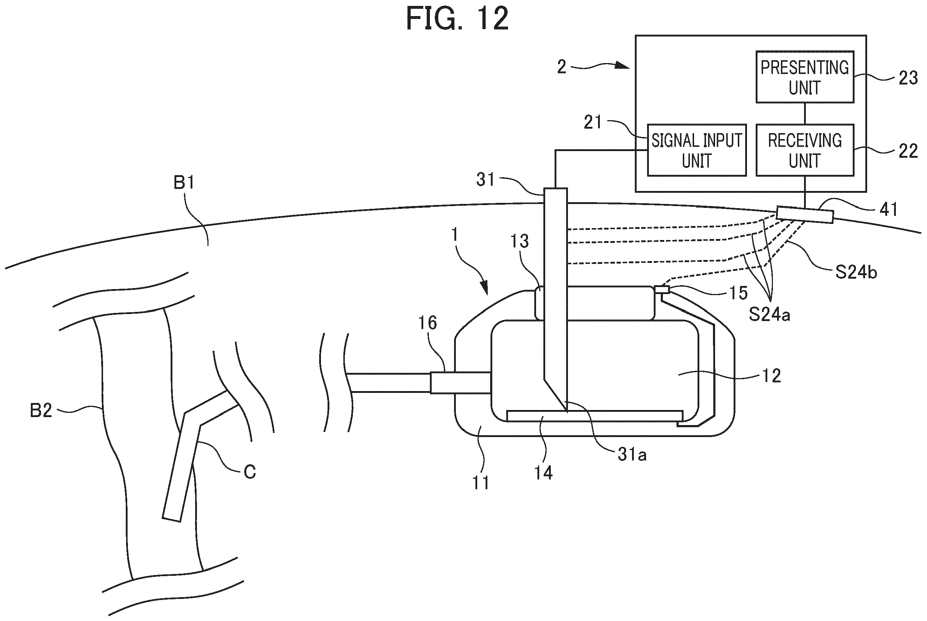

The level L25 shown in FIG. 8 indicates the level of the electric signal when the distal end portion 31a of the injection needle 31 comes into contact with the first conductive portion 14. FIG. 12 is a diagram schematically showing a representative example of paths S24a and S24b. The paths S24a and S24b refer to paths through which electric signals inputted to the injection needle 31 are transmitted to the third conductive portion 41 when the distal end portion 31a of the injection needle 31 comes into contact with the first conductive portion 14.

The determination unit 25 refers to the data stored in the storage unit 27 based on the level of the electric signal received by the receiving unit 22. Then, the determination unit 25 determines whether or not the distal end portion of the injection needle 31 is included in the container 12. The storage unit 27 stores, for example, data of the waveform shown in FIG. 8.

More specifically, when the electric signal received by the receiving unit 22 indicates the level L21, the determination unit 25 determines that the injection needle 31 is located outside the body and that the distal end portion of the injection needle 31 is not included in the container 12.

When the electric signal received by the receiving unit 22 is the level L22, the determination unit 25 determines that the distal end portion 31a of the injection needle 31 is inserted into the living body B1 and the distal end portion 31a of the injection needle 31 is not included in the container 12.

When the electric signal received by the receiving unit 22 is the level L23, the determination unit 25 determines that the distal end portion 31a of the injection needle 31 is inserted into the soft portion 13 and the distal end portion 31a of the injection needle 31 is not included in the container 12.

When the electric signal received by the receiving unit 22 is the level L24, the determination unit 25 determines that the distal end portion 31a of the injection needle 31 is inserted into the container 12.

When the electric signal received by the receiving unit 22 is the level L25, the determination unit 25 determines that the distal end portion 31a of the injection needle 31 is in contact with the first conductive portion 14 disposed on the bottom surface of the container 12.

It should be noted that the determination unit 25 may determine whether the distal end portion of the injection needle 31 is included in the container 12 or not based on the difference (change) between the levels.

Also, amplitude A, amplitude B, amplitude C, and amplitude D are assumed to be different, respectively. The amplitude A refers to the amplitude of the electric signal when the injection needle 31 to which the electric signal is inputted passes through the living body B1. The amplitude B refers to the amplitude of the electric signal when the injection needle 31 passes through the soft portion 13. The amplitude C refers to the amplitude of the electric signal when the injection needle 31 passes through the liquid in the container 12. The amplitude D refers to the amplitude of an electric signal when the distal end portion 31a of the injection needle 31 contacts the first conductive portion 14. Therefore, the determination unit 25 may determine whether the distal end portion of the injection needle 31 is included in the container 12 or not based on the amplitude of the electric signal, instead of the determination based on the level of the electric signal. Furthermore, the determination unit 25 may determine whether the distal end portion of the injection needle 31 is included in the container 12 or not based on the change in the amplitude of the electric signal. For example, when the amplitude of the electric signal changes from the amplitude A to the amplitude D (the amplitude A → amplitude B → the amplitude C → amplitude D), the determination unit 25 may determine that the distal end portion of the injection needle 31 is included in the container 12.

Furthermore, the determination unit 25 may determine whether the distal end portion of the injection needle 31 is included in the container 12 or not based on information other than the amplitude of the electric signal. For example, an element for converting an electric signal may be provided between the first conductive portion 14 and the second conductive portion 15. This element is, for example, an IC chip or an LED. In the case of such a configuration, the element changes the frequency of the electric signal transmitted to the external electrode, changes the pulse interval, or superimposes predetermined information based on the received amount of current. The determination unit 25 may determine whether the distal end portion of the injection needle 31 is included in the container 12 or not based on the frequency, the pulse interval, and the superimposed information.

Furthermore, the medical device 1 may include a light emitting portion (not shown) or the like. In such a configuration, the second conductive portion 15 and the light emitting portion are connected to each other, and when the electric signal is transmitted to the second conductive portion 15, the light emitting portion emits light. The user can confirm light emission by the light emitting portion from the inside of the body. Thus, the user can determine whether or not the distal end portion of the injection needle 31 is included in the container 12. It should be noted that the light emission of the light emitting portion may be simply illuminated. The light emitting portion may change the emission color. Alternatively, the light emitting portion may emit light in a predetermined blinking pattern.

It should be noted that the descriptions are given of whether or not the position of the distal end portion 31a of the injection needle 31 is located outside the body, is located in the living body B1, is located in the soft portion 13, and is included in the container 12. Furthermore, the descriptions are given of whether or not the distal end portion 31a of the injection needle 31 is in contact with the first conductive portion 14. However, the present disclosure is not limited thereto. At least when the distal end portion 31a of the injection needle 31 comes into contact with the first conductive portion 14, the path is shortest and the level of the electric signal reaches a maximum. Further the level of the electric signal becomes constant while the soft portion 13 is punctured. Accordingly, the location of the distal end portion 31a of the injection needle 31 may be determined by these two states.

### (Second Embodiment)

### <Configuration and Operation of Signal Processing Device according to Second Embodiment>

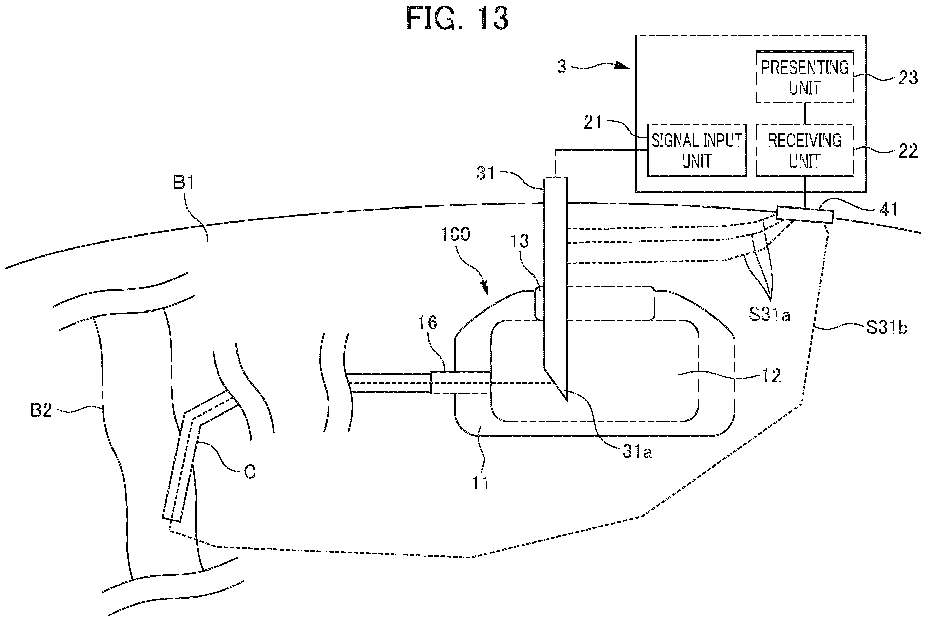

Next, a description will be given of the configuration and operation of the signal processing device according to the second embodiment. FIG. 13 is a schematic diagram showing a schematic configuration of a medical device 100 and a signal processing device 3 according to the second embodiment. In the second embodiment, as shown in FIG. 13, the configuration of the medical device is different from the first embodiment. In the second embodiment, the medical device 100 does not include the first conductive portion 14 and the second conductive portion 15. That is, the medical device 100 according to the second embodiment does not involve any additional change from an existing device. Furthermore, in the following description, the same components as those of the medical device 1 and the signal processing device 2 according to the first embodiment are denoted by the same reference numerals, and detailed descriptions thereof are omitted.

The signal processing device 3 includes a signal input unit 21, a receiving unit 22, and a presenting unit 23, as shown in FIG. 13. The signal input unit 21 inputs an electric signal to a conductive injection needle 31. The receiving unit 22 receives an electric signal outputted from the distal end portion 31a of the injection needle 31 inserted into the body via the electrically conductive third conductive portion 41 disposed at a predetermined location outside the body. The presenting unit 23 presents information based on the electric signal received by the receiving unit 22.

FIG. 13 shows a state in which the distal end portion 31a of the injection needle 31 is inserted into the container 12. The container 12 and the catheter C are filled with a portion of a saline solution, a portion of blood, or the like. In addition, the saline solution and the blood have electrical conductivity.

According to such a configuration, when the injection needle 31 is inserted into the living body B1 by the user, and the distal end portion 31a of the injection needle 31 passes through the soft portion 13 and reaches the container 12, as shown in FIG. 13, the electric signal outputted from the distal end portion 31a is transmitted via the injection part 16, the catheter C, and the blood vessel B2 into the living body B1 and to the third conductive portion 41. It should be noted that, in FIG. 13, the path in the living body B1 is shown by the paths S31a and S31b. However, this path is merely an example. The present disclosure is not limited to this path. The signal processing device 3 receives the electric signal transmitted to the third conductive portion 41 by the receiving unit 22. The signal processing device 3 presents information based on the received electric signal by the presenting unit 23.

Therefore, it is possible for the signal processing device 3 to present information as to whether the distal end portion 31a of the injection needle 31 is included in the container 12 of the medical device 100 or not to the user who punctures with the injection needle 31 the medical device 100 having the same configuration as the existing device.

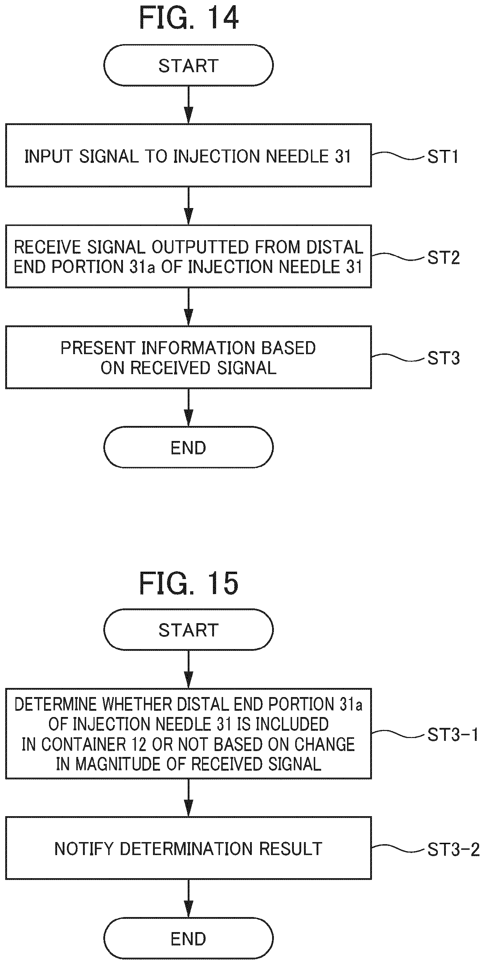

Next, a signal processing method will be described. The signal processing method includes using the signal processing device 2 to present information relating to whether the injection needle 31 is included in the container 12 of the medical device 1 or not. FIG. 14 is a flowchart for explaining the signal processing method. It should be noted that, in the following, a description will be given of a signal processing method using the signal processing device 2. However, a signal processing method using the signal processing device 3 is the same as the signal processing method using the signal processing device 2.

In step ST1, the signal input unit 21 inputs an electric signal to the conductive injection needle 31 (signal inputting step). For example, the signal input unit 21 and the injection needle 31 are electrically connected to each other by a sterilized clip or the like. The signal processing devices 2 and 3 input the electric signal from the signal input unit 21 to the injection needle 31 in accordance with the operation of the user. The user punctures with the injection needle 31 the soft portion 13 of the medical device 1 implanted in the subject's body.

In step ST2, the receiving unit 22 receives the electric signal outputted from the distal end portion 31a of the injection needle 31 (receiving step). The path through which the electric signal outputted from the distal end portion 31a of the injection needle 31 passes through the living body B1 and is received by the receiving unit 22 differs depending on the position of the injection needle 31. The paths include, for example, the path S21 (see FIG. 9), the path S22 (see FIG. 10), the paths S23a and S23b (see FIG. 11), and the paths S24a and S24b (see FIG. 12).

In step ST3, the presenting unit 23 presents information based on the electric signal received in step ST2 (presenting step). Step ST3 further includes the following determining step and notifying step. FIG. 15 is a flowchart for explaining the presenting step according to step ST3.

In step ST3-1, the storage unit 27 stores data indicating a change in the electric signal. This data shows the change in the electric signal during the time when the injection needle 31, to which the electric signal is inputted by step ST1, passes through the soft portion 13 of the medical device 1 and reaches the inside of the container 12. In step ST3-1, the determination unit 25 refers to the data, and determines whether the distal end portion 31a of the injection needle 31 is included in the container 12 or not based on the change in the magnitude of the electric signal measured in step ST2.

In step ST3-2, the notification unit 26 notifies the determination result by step ST3-1.

According to such a configuration, it is possible for the signal processing device 2 to present information relating to whether the distal end portion 31a of the injection needle 31 is included in the container 12 of the medical device 1 or not to a user who punctures the medical device 1 with the injection needle 31.

### (Signal Processing Program)

A signal processing program that presents information relating to puncture of an injection needle with respect to a medical device mainly includes the following steps, and is executed by the computer 500 (hardware).

Step 1 (signal inputting step): inputting an electric signal to the conductive injection needle 31. Step 2 (receiving step): receiving an electric signal outputted from the distal end portion 31a of the injection needle 31. Step 3 (presenting step): presenting information based on the electric signal received in Step 2.

Furthermore, step 3 (presenting step) includes the following steps, and is executed by the computer 500 (hardware).

Step 3-1 (determining step): referring to data which is stored in the storage unit 27 and indicates a change in the electric signal during the time when the injection needle 31, to which an electric signal is inputted by Step 1, passes through the soft portion 13 of the medical device 1 and reaches the inside of the container 12, and determining whether the distal end portion 31a of the injection needle 31 is included in the container 12 or not based on the change in the magnitude of the electric signal measured in Step 2. Step 3-2 (notifying step): notifying the result of the determination in Step 3-1.

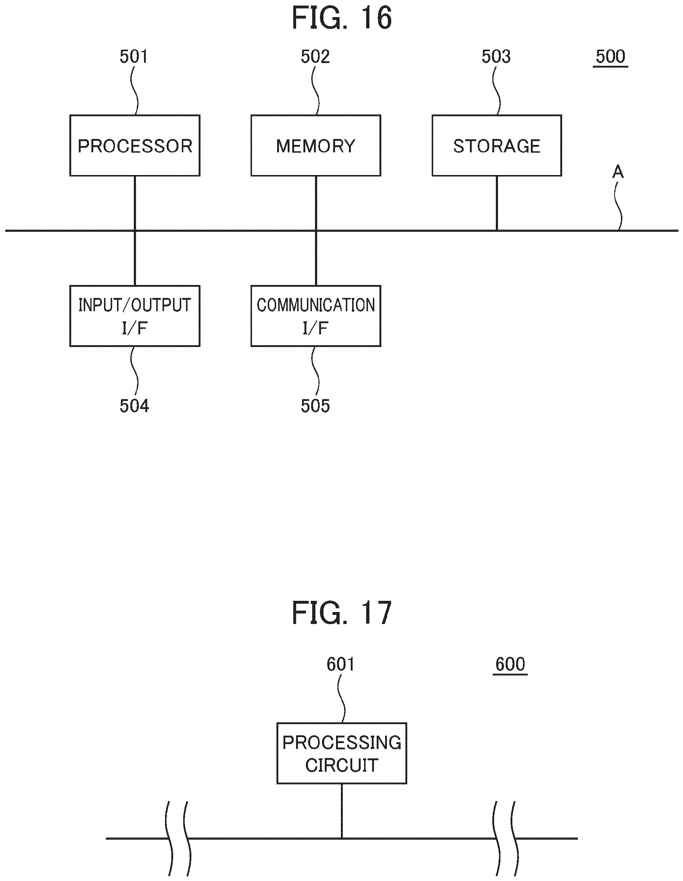

Here, the configuration and the operation of the computer 500 will be described with reference to the drawings. FIG. 16 is a diagram showing a configuration of the computer 500. As shown in FIG. 16, the computer 500 includes a processor 501, a memory 502, a storage 503, an input/output I/F 504, and a communication I/F 505. These components are connected on a bus A. Each of these components cooperates with each other, thereby realizing the functions and/or methods described in the present disclosure.

For example, a display and a touch panel are connected to the input/output I/F 504. The display shows various kinds of information. The touch panel receives operations by a user. The touch panel is placed in front of the display. Therefore, the user can perform intuitive operation by touching icons displayed on the display with a finger or the like. It should be noted that the touch panel may not be disposed on the front surface of the display. Furthermore, instead of the touch panel, or together with the touch panel, a keyboard and a pointing device such as a mouse may be connected to the input/output I/F 504. A speaker and a microphone may be connected to the input/output I/F 504. The speaker may output sound to the outside. External audio may be inputted to the microphone.

The display includes, for example, a liquid crystal display and an organic electro luminescence display. The display displays various information under control of the processor 501. The processor 501 controls the display based on the signal processing program. This realizes the function of the notification unit 26.

The memory 502 includes an RAM (Random Access Memory). The RAM may include volatile or non-volatile memory.

The storage 503 includes an ROM (Read Only Memory). The ROM includes non-volatile memory. The ROM is realized by, for example, an HDD (Hard Disc Drive) or an SSD (Solid State Drive). The storage 503 corresponds to the storage unit 27 described above. The storage 503 stores various programs such as the signal processing programs realized in the above-described steps 1 to 3 and steps 3-1 and 3-2.

For example, the processor 501 controls the overall operation of the computer 500. The processor 501 is an arithmetic unit. The processor 501 loads various programs from the storage 503 to the memory 502 for implementing an operating system and various functions. The processor 501 executes instructions included in the loaded program.

More specifically, when the operation by the user is accepted, the processor 501 reads a program (e.g., a signal processing program) stored in the storage 503. The processor 501 expands the read program into the memory 502 and executes the program. Furthermore, the processor 501 executes the signal processing program. As a result, the functions of the presenting unit 23 and the determination unit 25 are realized.

Here, the configuration of the processor 501 will now be described. The processor 501 may be implemented, for example, by a CPU (Central Processing Unit), an MPU (Micro Processing Unit), a GPU (Graphics Processing Unit), various other arithmetic units, or combinations thereof.

Furthermore, in order to realize the functions and/or methods described in the present disclosure, part or all of the functions such as the processor 501, the memory 502, and the storage 503 may be configured by a processing circuit 601, which is a dedicated hardware, as shown in FIG. 17. FIG. 17 is a diagram showing a configuration of the processing circuit 601 of the computer 600. The processor circuit 601 may be, for example, a single circuit, a complex circuit, a programmed processor, a parallel programmed processor, an ASIC (Application Specific Integrated Circuit), a FPGA (Field-Programmable Gate Array), or combinations thereof.

Furthermore, although the processor 501 has been described as a single component, the present disclosure is not limited to this, and the processor 501 may be composed of a plurality of physically separate sets of processors. In the present disclosure, programs described as being executed by the processor 501 or instructions included in the programs may be executed by a single processor 501 or may be executed by a plurality of processors in a distributed manner. Furthermore, the programs executed by the processor 501 or the instructions included in the programs may be executed by a plurality of virtual processors.

In this manner, the signal processing program is executed by the computers 500 and 600. Thus, it is possible to present information relating to whether the distal end portion 31a of the injection needle 31 is included in the container 12 of the medical device 1 or not to a user who punctures the medical device 1 with the injection needle 31.

While some embodiments of the present application have been described in detail based on the drawings, these are illustrative, and it is possible to implement the present disclosure in other forms which have been subjected to various modifications and improvements based on the knowledge of those skilled in the art, including the aspects described in the sections of disclosure of the present disclosure.

### EXPLANATION OF REFERENCE NUMERALS

- 1,100: medical device
- 2,3: signal processing device
- 11: main body
- 12: container
- 12a: opening
- 13: soft portion
- 14: first conductive portion
- 15: second conductive portion
- 16: injection part
- 21: signal input unit
- 22: receiving unit
- 23: presenting unit
- 31: injection needle
- 31a: distal end portion
- 41: third conductive portion

## Claims

1. A medical device used for implanting in a body, comprising:
a main body;
a container included in the main body;
an insulating soft portion that closes an opening of the container;
an electrically conductive first conductive portion disposed on a bottom of the container; and
an electrically conductive second conductive portion disposed on the main body, wherein
the first conductive portion and the second conductive portion are electrically connected to each other.

2. The medical device according to claim 1, further comprising an injection part to which a catheter is connected, the injection part being configured to inject a liquid in the container into a blood vessel through the catheter.

3. The medical device according to claim 1 or 2, wherein the second conductive portion has a rectangular shape, an annular shape, or a partially notched curved shape.

4. A signal processing device comprising:
a signal input unit that inputs an electric signal to an electrically conductive injection needle;
a receiving unit that receives an electric signal outputted from a distal end portion of the injection needle inserted into a body via an electrically conductive third conductive portion disposed at a location on a body surface, the location being determined based on a position of the second conductive portion of the medical device according to any one of claims 1 to 3; and
a presenting unit that presents information based on the electric signal received by the receiving unit.

5. The signal processing device according to claim 4, wherein the presenting unit presents a magnitude of the electric signal received by the receiving unit.

6. The signal processing device according to claim 4, wherein the receiving unit receives, via the second conductive portion, an electric signal generated when the injection needle and the first conductive portion are electrically connected to each other.

7. The signal processing device according to claim 4,
wherein the presenting unit includes
a determination unit that determines whether the distal end portion of the injection needle is included in the container or not based on the electric signal received by the receiving unit, and
a notification unit that notifies a determination result by the determination unit.

8. The signal processing device according to claim 7, further comprising a storage unit that stores data indicating a change in an electric signal during a time when the injection needle, to which the electric signal is inputted by the signal input unit, enters a body from an outside of the body, passes through the soft portion, and reaches an inside of the container,
wherein the determination unit refers to the data stored in the storage unit, and determines whether the injection needle is included in the container or not based on the electric signal received by the receiving unit.

9. The signal processing device according to claim 8, wherein the data stored in the storage unit includes a change in a magnitude of an electric signal until the injection needle reaches inside the container and the distal end portion of the injection needle comes into contact with the first conductive portion.

10. A signal processing device comprising:
a signal input unit that inputs an electric signal to an electrically conductive injection needle;
a receiving unit that receives an electric signal outputted from a distal end portion of the injection needle inserted into a body via an electrically conductive third conductive portion disposed at a predetermined location outside the body; and
a presenting unit that presents information based on the electric signal received by the receiving unit.

11. A signal processing method comprising:
a signal inputting step including inputting an electric signal to an electrically conductive injection needle;
a receiving step including receiving an electric signal outputted from a distal end portion of the injection needle; and
a presenting step including presenting information based on the electric signal received in the receiving step.

12. The signal processing method according to claim 11, the presenting step further includes
a determining step including referring to data which is stored in a storage unit and indicates a change in an electric signal during a time when the injection needle, to which the electric signal is inputted by the signal inputting step, passes through a soft portion of a medical device and reaches inside of a container, and determining whether the injection needle is included in the container or not based on a change in a magnitude of the electric signal measured in the receiving step, and
a notifying step including notifying a determination result by the determining step.

13. A signal processing program for executing a computer, comprising:
a signal inputting step including inputting an electric signal to an electrically conductive injection needle;
a receiving step including receiving an electric signal outputted from a distal end portion of the injection needle; and
a presenting step including presenting information based on the electric signal received in the receiving step.
